# EUROPEAN PATENT APPLICATION

(11) **EP 1 964 550 A1**
(43) Date of publication of application: **03.09.2008**
(21) Application number: 07004225.4
(22) Date of filing: 01.03.2007
(51) Int. Cl.: A61K 31/00, A61K 31/57, A61K 31/58, A61K 45/06, A61P 17/00

(54) **Glucocorticoid-nitrooxyderivative compositions**

(71) Applicant: NicOx S.A., 06560 Sophia Antipolis - Valbonne (FR); FERRER INTERNACIONAL, S.A., 08028 Barcelona (ES)
(72) Inventor: Benedini, Francesca, 20097 San Donato Milanese (Milano) (IT); Ongini, Ennio, 20090 Segrate (Milano) (IT); Bolla, Manlio, 06130 Grasse (FR); Guglietta, Antonio, 08750 Molins de Rei (Barcelona) (ES); Palop, Daniel, 08390 Mongat (ES); Princep, Marta, 08028 Barcelona (ES)
(74) Representative: Barchielli, Giovanna

(57) **Abstract**

The invention relates to compositions comprising a glucocorticoid-nitrooxyderivative and at least an active ingredient, to topical pharmaceutical formulations thereof. The invention also relates to the use for treating skin or mucosal membrane diseases or disorders. These compositions have an improved pharmacological activity and enhanced local tolerability.

## Description

The present invention relates to compositions comprising a glucocorticoid-nitrooxyderivative and at least a further active compound, to topical pharmaceutical formulations thereof. These compositions are used for treating skin or mucosal membrane diseases or disorders.

Most of the skin or mucosal membrane diseases or disorders are the result of inflammation caused by inflammatory agents, such as, but not limited to, bacterial, fungal, viral, parasitic, autoimmune, allergic, hormonal and/or malignant inflammatory agents. The most common skin diseases or disorders include, but is not limited to, corticosteroid-responsive dermatosis, atopic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch and ruber lichen planus. Dermatitis and eczema result from inflammatory processes that involve the upper dermis and epidermis of the skin. When eczema develops, the keratinocytes in the epidermis distend from one another and fluid is accumulated there amongst in a process known as spongiosis.
In chronic forms of eczema or dermatitis the main change include thickening of the epidermis, which leads to itching, roughening and scaling of the skin surface. The loss of water from the skin leads to inflammation of the horny layer, which later results in cracked and sore skin.
Dermatitis is further classified into contact dermatitis (allergic or non allergic), atopic dermatitis and seborrheic dermatitis. Non-allergic contact dermatitis occurs in response to skin irritants, such as acids, alkalis, oils, detergents and solvents.
Allergic contact dermatitis occurs as a result of sensitization to repeated exposure to an antigen. Allergic contact dermatitis appears in skin areas that were in direct contact with the antigen.
Atopic dermatitis, which affects mainly infants, is characterized by sensitization of the skin to a wide range of common antigens.
Seborrheic dermatitis affects the scalp and other hairy areas, the face, and flexural areas and results from yeast or bacteria induced inflammation. Most people suffer from dandruff that is a mild form of seborrheic dermatitis. Psoriasis is a dominant autosomal inherited inflammatory disease characterized by enhanced proliferation of keratinocytes which proliferation leads to formation of scaly plaques on, for example, the knees, elbows, buttocks,
and which are aesthetically unpleasant and cause discomfort to the affected subject.
Topical corticosteroids are a powerful tool for treating skin disease.
In clinical practice, for example the use of super potent topical steroids is typically limited to only two weeks because of their use may be associated with adverse side effects such as skin atrophy, burning, itching, irritation, dryness, folliculitis, hypertrichosis, acne, hypo pigmentation, perioral dermatitis, allergic contact dermatitis, maceration of the skin, and secondary infection.

Although topical administration of corticosteroids minimizes the side-effects as compared to systemic administration, the active compounds are still absorbed into the circulation where they are systemically active. Systemic absorption of topical corticosteroids can result in reversible hypothalamic-pituitary-adrenal (HPA) axis suppression, Cushing's syndrome-like symptoms,
hyperglycemia, effects on bone growth in children and on bone density in the elderly, ocular complications (cataract formation and glaucoma) and skin atrophy.
Furthermore, tachyphylaxis may result from the use of the topical steroid.

A variety of topical compositions comprising a corticosteroid and another active compound such as an antibiotic, an antifungal or an anti-histamine agent have entered the marked, however these known treatments suffer from the severe and well known side effects associated with corticosteroids, or have not proved very effective.
There is, therefore, a need for compositions for dermatological application which are effective in treating skin or mucosal membrane diseases or disorders, yet have fewer side effects that the known treatments.

U.S. Pat. No. 4,335,121 discloses 6.alpha., 9.alpha.-Difluoro-17.alpha.-(1-oxopropoxy)-11.beta.-hydroxy-16.alpha.-meth yl-3-oxo-androsta-1,4-diene-17-0-carbothioic acid S-fluoromethyl ester (known by the generic name of fluticasone propionate) and derivatives thereof, these compounds have good anti-inflammatory activity, particularly on topical applications.
EP 0929565 discloses nitroxyesters of corticosteroids that among systemic uses can be used for the treatment of dermatological disorders; in particular the patent discloses nitroxyesters of corticosteroids in which the nitroxy group is covalently linked through an alkyl chain to the glucocorticoid moiety. The document reports that these nitroderivatives of steroids, after systemic administration, displayed enhanced efficacy and better systemic tolerability, such as better gastric tolerability and reduced cardiovascular side effects, compared with their parent compounds.

WO 03/064443 discloses nitrooxyderivatives of corticosteroids in which the nitrooxy group is covalently linked through an aromatic or a heteroarylic ring containing linker to the glucocorticoid moiety. The document reports that these nitrooxyderivatives of steroids, after systemic administration, displayed an improved pharmacological activity and lower side effect compared to their parent compounds. -

WO00/61604 discloses nitrooxyderivatives of corticosteroids in which the nitrooxy group is covalently linked through an "antioxidant moiety" to the glucocorticoid moiety, such "antioxidant moieties" are compounds capable to prevent the production of free radicals and are selected on the basis of tests described in the patent application. The document reports that these compounds can be used for the treatment of pathologies associated with an oxidative stress condition in which the corresponding parent compounds show lower activity or higher toxicity.
The above-mentioned documents do not disclose the nitrooxyderivatives of corticosteroids in combination with other therapeutic agents for treating skin disorders.

WO 97/34871 discloses nitrosated or nitrosylated steroids and their use for the treatment of respiratory disorders, in particular describes the activity in a pulmonary model of allergic asthma and lung inflammation of 9-fluoro-11β-hydroxy-16α,17α-[(1-methylethylidene)bis(oxy)] pregna-1,4-diene-3,20-dione-21(4-nitrooxy)-butanoate.
The document does not mention the use of the compounds in for the treatment of skin disorders.

Ilyun E. et al, British Journal of Pharmacology (2004) 143, 618-625, relates to a study of the activity of hydrocortisone 21-[4'-(nitrooxymethyl)benzoate] in a model of irritant acute dermatitis. In this study oedema formation and recruitment of leukocytes were evaluated and the results demonstrate that the compound has a higher anti-inflammatory activity than the parent compound hydrocortisone. The document does not report any information regarding the efficacy of hydrocortisone 21-[4'-(nitrooxymethyl)benzoate] in combination with other therapeutic agents.

The present invention solves the above-mentioned problems by providing compositions comprising a glucocorticoid nitrooxyderivative and at least an active compound selected from anti-bacterial agents, antifungal agents, antiviral agents, anti-inflammatory agents, anti-histamine agents, synthetic anti-acne agents, vitamins, retinoic acid metabolism-blocking agents, fusidic acid compounds, mupirocin compounds, salicylic acid compounds, aloe vera products, omega-3 fatty acid compounds, Aquifolium products, vegetal oils, urea compounds, minoxidil compounds and zinc compounds. These compositions show an improved pharmacologically profile, better pharmacokinetic and pharmacodynamic properties and fewer adverse side effects, in particular the compositions of the invention show an improved local tolerability, such as reduction of skin blanching and skin atrophy, a fast onset of action and an increased efficacy than the existing topical steroid formulations.

It is an object of the invention a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) at least a compound selected from an anti-bacterial agent, an antifungal agent, an antiviral agent, an anti-inflammatory agent, an anti-histamine agent, a synthetic anti-acne agent, a vitamin and a retinoic acid metabolism-blocking agent; and/or
   an active compound selected from a fusidic acid compound, a mupirocin compound, a salicylic acid compound, an aloe vera product, an omega-3 fatty acid compound, an Aquifolium product, a vegetal oil, a urea compound, a minoxidil compound and a zinc compound.

The component b) is preferably - selected among the following classes of anti-bacterial agents: quinolones, aminoglycosides, macrolides and tetracyclines or the component b) is preferably selected among the following classes of antifungal agents: imidazoles, allylamines and polyenes, or the component b) is preferably selected among antiviral agents, or the component b) is preferably selected among the following classes of anti-inflammatory agents: calcineurin inhibitors, capsaicin compounds and lactic acid compounds, or the component b) is preferably selected among anti-histamine agents, or the component b) is preferably selected among synthetic anti-acne agents, or the component b) is preferably selected among the following classes of vitamins: retinoids and vitamin D analogues, or the component b) is preferably selected among retinoic acid metabolism-blocking agents, or the component b) is preferably selected among fusidic acid and its pharmaceutically acceptable salts thereof; or Lhe component b) is preferably selected among mupirocin and its pharmaceutically acceptable salts thereof; or the component b) is preferably selected among salicylic acid and its pharmaceutically acceptable salts thereof; or the component b) is preferably an aloe vera product, more specifically an aloe vera extract; or the component b) is preferably selected among omega-3 fatty acid compounds, or the component b) is preferably an Aquafolium product, more specifically an extract of Aquafolium; or the component b) is preferably selected among vegetal oils, or the component b) is preferably an urea compound, more specifically urea; or the component b) is preferably a minoxidil compound, more specifically minoxidil; or the component b) is preferably selected among zinc compounds, more specifically zinc oxide, zinc salts and zinc pyrithione.

A preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-(4-(nitrooxymethyl-)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) an anti-bacterial agent selected from the group consisting of a quinolone, an aminoglycoside, a macrolide and a tetracycline.

Another preferred embodiment of the present invention relates to a composition comprising:
a) a glucocorticoid nitrooxyderivative selected from:
   - (110,16a)-9-fluoro-11-hydroxy-16,17-[l-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxyniethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) an antifungal agent selected from the group consisting of an imidazole, an allylamine and a polyene.Another preferred embodiment of the present invention relates to a composition comprising:
   a) a glucocorticoid nitrooxyderivative selected from:
      - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
      - (11β-17-[(ethoxycarbonyl)oxy]-11-hydroxy-21-[1-oxo-[4-(nitrooxymethyl)benzoxy]]pregna-1,4-diene-3,20-dione; or
      - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
   b) an anti-inflammatory agent selected from the group consisting of a calcineurin inhibitor, a capsaicin compound and a lactic acid compound.

Another preferred embodiment of the present invention relates to a composition comprising:
a) a glucocorticoid nitrooxyderivative selected from:
   - (11β,16α) -9-fluoro-11-hydroxy-16,17-[1-methylethylide nebis(oxy)1-21-[1-oxo-[4-(nitrooxymethyl)benzoxy]] pregna-1,4-diene-3,20-dione;
   - (11β)-17-[(ethoxycarbonyl)oxyl-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) a vitamin selected from the group consisting of a retinoid and a vitamin D analogue.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3, 20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) a fusidic acid compound which is selected from fusidic acid and pharmaceutically acceptable salts thereof.

Another preferred embodiment of the present invention relates to a composition comprising:
a) a glucocorticoid nitrooxyderivative selected from:
   - (11β,16α)-9-fluoro-11-hydroxy-16,17-[17methylethylide nebis(oxy)]-21-[1-oxo-[4-(nitrooxymethyl)benzoxy)] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,26β)-9-fluoro-11-hydroxy-16-methyl-21-[1-oxo-[4-(nitrooxymethyl)benzoxy]]-17-(valeryloxy)pregna-1,4-diene-3,20-dione; and
b) a mupirocin compound which is selected from mupirocin and pharmaceutically acceptable salts thereof.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) a salicylic acid compound which is salicylic acid.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-dicne-3,20-dione; and
b) an aloe vera product which is an aloe vera extract.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) an omega-3 fatty acid compound which is selected from docosahexaenoic acid and pharmaceutically acceptable esters thereof, eicosapentaenoic acid and pharmaceutically acceptable esters thereof, and linolenic acid and pharmaceutically acceptable esters thereof.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-2l-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) an Aquafolium product which is an extract of Aquafolium.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11(β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) an urea compound which is urea.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-(1-oxo-[4-(nitrooxymethyl)benzoxy]] pregna-1,4-diene-3,20-dione;
   - (11β)-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxyl]-17-(valeryloxy)pregna-1,4-diene-3,20-dione; and
b) a minoxidil compound which is minoxidil.

Another preferred embodiment of the present invention relates to a composition comprising:
a) a glucocorticoid nitrooxyderivative selected from:
   - (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione;
   - (11β-17-[(ethoxycarbonyl)oxy]-11-hydroxy-21-[1-oxo-[4-(nitrooxymethyl)benzoxy]]pregna-1,4-diene-3,20-dione; or
   - (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) a zinc compound which is selected from zinc oxide, pharmaceutically acceptable zinc salts and zinc pyrithione.

A preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)ben2oxy)] pregna-1,4-diene-3,20-dione; and
b) an anti-bacterial agent selected from the group consisting of a quinolone, an aminoglycoside, a macrolide and a tetracycline.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an antifungal agent selected from the group consisting of an imidazole, an allylamine and a polyene.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an anti-inflammatory agent selected from the group consisting of a calcineurin inhibitor, a capsaicin compound and a lactic acid compound.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-dierre-3,20-dione: and
b) a vitamin selected from the group consisting of a retinoid and a vitamin D analogue.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]1 pregna-1,4-diene-3,20-dione; and
b) a fusidic acid compound which is selected from fusidic acid and pharmaceutically acceptable salts thereof.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) a mupirocin compound which is selected from mupirocin and pharmaceutically acceptable salts thereof.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) a salicylic acid compound which is salicylic acid.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an aloe vera product which is an aloe vera extract.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an omega-3 fatty acid compound which is selected from docosahexaenoic acid and pharmaceutically acceptable esters thereof, eicosapentaenoic acid and pharmaceutically acceptable esters thereof, and linolenic acid and pharmaceutically acceptable esters thereof.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an Aquafolium product which is an extract of Aquafolium.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) an urea compound which is urea.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) a minoxidil compound which is minoxidil.

Another preferred embodiment of the present invention relates to a composition comprising:
a) (11β,16α)-9-fluoro-11-hydroxy-16,17-[1-methylethylide nebis(oxy)]-21-[1-oxo-[4-(nitrooxymethyl)benzoxy]] pregna-1,4-diene-3,20-dione; and
b) a zinc compound which is selected from zinc oxide, pharmaceutically acceptable zinc salts and zinc pyrithione.

The combination of a glucocorticoid nitrooxyderivative and at least a further active component has shown a synergic effect which makes it possible to use lower doses of the individual components which allows to reduce the side effects and, therefore, to improve the compliance of the patients.

The compositions of the present invention are useful for the treatment of skin or mucosal membrane diseases or disorders. Such conditions comprise, but are not limited to, corticosteroid-responsive dermatosis, atopic dermatitis, contact dermatitis, seborrheic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch, ruber lichen planus, and seborrheic dermatitis which affects the scalp and other hairy areas.
The compositions of the present invention are particularly useful for the treatment of corticosteroid-responsive dermatosis, atopic dermatitis, contact dermatitis, psoriasis and seborrheic dermatitis.

Skin or mucosal membrane diseases or disorders comprise, but are not limited to, corticosteroid-responsive dermatosis, atopic dermatitis, contact dermatitis, seborrheic dermatitis, inflammation, eczema, erythema, papulation, scaling, erosion, oozing, crusting, pruritis, psoriasis, epidermalysis bullosa, erythema, hidradenitis suppurative, warts, diaper rash, jock itch and ruber lichen planus.

Also within the scope of the invention are pharmaceutical formulations suitable for topical administration comprising a composition of the present invention and at least an excipient.
Preferred pharmaceutical dosage forms include cream, lotion and ointment formulation or topical spray compositions.
The pharmaceutical dosage forms are prepared according to procedures well known in the art.
The amount of the composition of formula the present invention in the topical formulation depends on the precise type of formulation to be prepared but it is generally within the range of around 0.001-12% by weight, more preferably 0.001 to 10% by weight. Generally, however for most types of preparations advantageously the amount is within the range of from 0.001 to 1% by weight, more preferably 0.01-0.5%, and especially around 0.025 to 0.1%.
Various optional ingredients may also be present- in the topical formulations and these are: one or more various solvents such as various short chain alcohols including, but not limited to, ethyl alcohol, propylene glycol, triacetin, hexylene glycol, and combinations thereof; suitable occlusive agents that may be present in the topical formulation include, but are not limited to, petrolatum, microcrystalline wax, dimethicone, beeswax, mineral oil, squalane, liquid paraffin, shea butter, carnauba wax, SEPIGEL.RTM. (a blend of isoparaffin/polyacrylamide- /laureth-7), and combinations thereof; surfactant such as, but are not limited to, CETOMACROGOL.RTM. 1000, (Crodor, Inc.) glycerol monostearate, glycerol distearate, glyceryl stearate, polyoxyethylene stearate, a blend of glyceryl stearate and PEG-100 stearate (as ARLACEL 165), polysorbate 40, polysorbate 60, polysorbate 80, CETETH-20.RTM., sorbitan monopalimate, sorbitan monostearate, sorbitan monooleate, and combinations thereof. Other various optional ingredients may also be present in the topical formulation. These are carriers (such as water or mineral oil), skin conditioners (such as lanolin, glycerine, cholesterol, cetostearyl alcohol, dimethicone PEG 100, PEG 200, PEG 300, PEG 400 or isopropylmyristate), buffers (such as sodium citrate/citric acid, dibasic sodium phosphate/citric acid, or monobasic sodium phosphate/citric acid), or preservatives (such as imidurea, methylparaben, or propylparaben).

### Experimental part

### Preparations of the compounds of the group a)

### Example 1

### Synthesis of (11β,16α)-9-fluoro-11-hydroxy-16,17-[1-methyl ethylidenebis(oxy)]-21-[1-oxo-[4-(nitrooxymethyl)benzoxy]] pregna-1,4-diene-3,20-dione of formula (I)

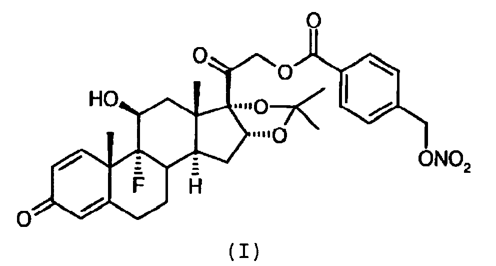

To a solution of triamcinolone acetonide (2.47 g, 5.7 mmol) in dichlorometane (55 ml), 4-(nitrooxymethyl)benzoic acid (1.38 g, 7.0 mmol), DMAP (0.07 g, 0.54 mmol) and EDAC (1.39 g, 7.2 mmol) were added. The reaction was stirred at room temperature for 24 hours. The solution was treated with water, the organic layers were dried with sodium sulfate and concentrated under reduced pressure. The residue was purified by flash chromatography, eluent dichloromethane/ethyl acetate 95/5. The product (1.2 g) was obtained as white powder.
¹H-NMR (DMSO) δ: 8.05 (2H, d); 7.64 (2H, d); 7.29 (1H, d); 6.23 (1H, dd); 6.01 (1H, s); 5.68 (2H, s); 5.52 (1H, d); 5.42 (1H, d); 5.01 (1H, d); 4.86 (1H, d); 4.2 (1H, bs); 2.7-2.25 (4H, m); 2.15-1.72 (4H, m); 1.65-1.45 (5H, m); 1.36 (3H, s); 1.21 (3H, s); 0.87 (3H, s).

### Example 2

### Synthesis of (11β)-17-[(ethoxycarbonyl)oxyl-11-hydroxy-21-[1-oxo-[4-(nitrooxy methyl)benzoxy]]pregna-1,4-diene-3,20-dione (formula (II))

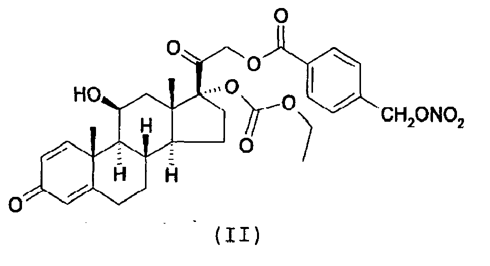

To a solution of prednisolone 17-ethylcarbonate (1.77 g, 4.1 mmol) in dichlorometane (40 ml), 4-(nitrooxymethyl)benzoic acid (1.0 g, 5.0 mmol), DMAP (0.05 g, 0.41 mmol) and EDAC (1.0 g, 5.2 mmol) were added. The reaction was stirred at room temperature for 24 hours. The solution was treated with water, the organic layers were dried with sodium sulfate and concentrated under reduced pressure. The residue was purified by flash chromatography, eluent n-hexane/ethyl acetate 6/4. The product (0.47 g) was obtained as white powder by crystallization with n-hexane/ethylacetate. m.p. = 113-119°C
¹H-NMR (DMSO) δ: 8.07 (2H, d) ; 7.66 (2H; d); 7.32 (1H, dd); 6.18 (1H, dd); 5.93 (1H, s); 5.70 (2H, s); 5.15 (2H, m); 4.90 (1H, d); 4.33 (1H, m); 4.12 (2H, m); 2.80-2.76 (1H, m); 2.56-2.50 (1H, m); 2.32-2.28 (1H, m); 2.11-1.99 (1H, m); 1.90-1.78 (4H, m); 1.6-1.36 (5H, m); 1.25-1.15 (4H, m); 1.10-0.9 (5H, m).

### Example 3

### Synthesis of (11β,16β)-9-fluoro-11-hydroxy-16-methyl-21-[1-oxo-[4-(nitrooxymethyl)benzoxy]]-17-(valeryloxy)]-pregna-1,4-diene-3,20-dione (formula (III))

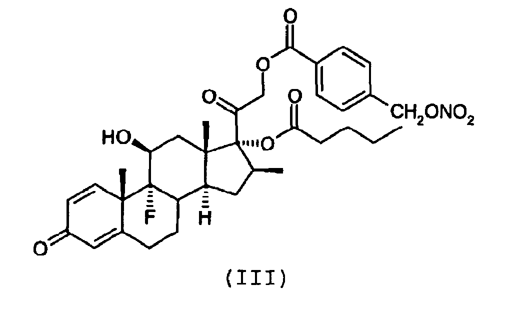

To a solution of betamethasone-17-valerate (2.54 g, 5.3 mmol) in dichlorometane (50 ml), 4-(nitrooxymethyl)benzoic acid (1.3 g, 6.5 mmol), DMAP (0.065 g, 0.53 mmol) and EDAC (1.53 g, 8.0 mmol) were added. The reaction was stirred at room temperature for 4 hours. The solution was treated with water, the organic layers were dried with sodium sulfate and concentrated under reduced pressure. The residue was purified by flash chromatography, eluent n-hexane/ethyl acetate 65/35. The product (0.72 g) was obtained as white powder by crystallization with n-hexane/ethylacetate.
m.p.=158-160°C
¹H-NMR (DMSO) δ: 8.03 (2H, d); 7.63 (2H, d); 7.29 (1H, d); 6.24 (1H, dd); 6.02 (1H, s); 5.68 (2H, s); 5.6 (1H, d); 4.97 (1H, d); 4.71 (1H, d); 4.24 (1H, m); 2.7-2.2 (4H, m); 2.15-1.75 (6H, m); 1.58- 1.05 (13H, m); 0.9 (3H, s); 0.85 (3H, t).
The compounds of the group b) are commercially available compounds or they can be prepared by well known methods.

### Evaluation of the pharmacological activity

The compositions of the present invention tested in different animal models have shown an enhanced efficacy in the treatment of dermatitis and a better effectiveness in reducing the inflammation than the known combinations.
The performed tests are:

### 1a) In vivo determination of inhibition of TPA-induced ear oedema after topical administration of test compounds.

The tests were performed according to the methods described by Carlson et al., Agents Actions 17:197-204, 1985, and Lucas et al., J Pharmacol Exp Ther 304:1172-1180, 2003.

Groups of 5-9 male Swiss mice of 27±5 g were used. Inflammation dermatitis was induced by applying 2 µg/ear of TPA (Tetradecanoyl Phorbol Acetate) dissolved with ethanol absolute on the surface of either the dorsal aspect of both ears (20 µL/ear).
15 min before the application of TPA, mice received topically 20 µL of a solution of test compounds (0.39 nM in ethanol) per site applied directly on the skin of the left ear and the vehicle (ethanol 100%) on right ear. Vehicle-Vehicle treated mice were included as negative control group. Compounds were tested at equimolecular doses.
Animals were sacrificed at 3h or 5h post TPA dose. Equal sections of both ears were punched out immediately after and weighed. The percentage of change of the weight of left ear versus the weight of right ear was calculated for each animal, and the percentage of inhibition of change in weight of treated animals versus the change in weight of non-treated animals (negative control) was measured.

## Claims

1. A composition comprising:
a) a glucocorticoid nitrooxyderivative selected from:
- (11β,16α)-9-fluoro-11-hydroxy-16,17-[1-methylethylide nebis(oxy)]-21-[1-oxo-[4-(nitrooxymethyl)benzoxyl] pregna-1,9-diene-3,20-dione;
- (11β)-17-[(ethoxycarbonyl)oxyl-11-hydroxy-21-[1-oxo-[4-(nitrooxymethyl)benzoxy]]pregna-1, 4-diene-3,20-dione; or
- (11β,16β)-9-fluoro-11-hydroxy-16-methyl-21-[1-oxo-[4-(nitrooxymethyl)benzoxy]]-17-(valeryloxy)pregna-1,4-diene-3,20-dione; and
b) at least a compound selected from an anti-bacterial agent, an antifungal agent, an antiviral agent, an anti-inflammatory agent, an anti-histamine agent, a synthetic anti-acne agent, a vitamin and a retinoic acid metabolism-blocking agent; and/or
an active compound selected from a fusidic acid compound, a mupirocin compound, a salicylic acid compound, an aloe vera product, an omega-3 fatty acid compound, an Aquifolium product, a vegetal oil, a urea compound, a minoxidil compound and a zinc compound

2. A composition according to claim 1 wherein the anti-bacterial agent is selected from the group consisting of a quinolone, an aminoglycoside, a macrolide and a tetracycline.

3. A composition according to claim 1 wherein the antifungal agent is selected from the group consisting of an imidazole, an allylamine and a polyene.

4. A composition according to claim 1 wherein the anti-inflammatory agent is selected from the group consisting of a calcineurin inhibitor, a capsaicin compound and a lactic acid compound.

5. A composition according to claim 1 wherein the vitamin is selected from the group consisting of a retinoid and a vitamin D analogue.

6. A composition according to claims 1-5 wherein the corticosteroid is (11β,16α)-9-fluoro-11-hydroxy-16,17-[1-methylethylidenebis(oxy)]-21-[1-oxo-[4-(nitrooxymethyl)benzoxy]] pregna-1,4-diene-3,20-dione.

7. A composition according to any of claims 1 to 6 for use as medicament.

8. Use of a composition according to any of claims 1 to 6 for the preparation of drugs for treating skin or mucosal membrane diseases or disorders-which comprise eczema, erythema, papulation, scaling, erosion, oozing, crusting, pruritis, inflammation, epidermalysis bullosa, erythema, warts, diaper rash, jock itch and ruber lichen planus.

9. Use of a composition according to any of claims 1 to 6 for the preparation of drugs for treating corticosteroid-responsive dermatosis, inflammation, eczema, erythema, papulation, scaling, erosion, oozing, crusting, pruritis, epidermalysis bullosa, erythema, warts, diaper rash, jock itch, ruber lichen planus and seborrheic dermatitis.

10. Use of a composition according to any of claims 1 to 6 for the preparation of drugs for treating atopic dermatitis.

11. Use of a composition according to any of claims 1 to 6 for the preparation of drugs for treating contact dermatitis.

12. Use of a composition according to any of claims 1 to 6 for the preparation of drugs for treating psoriasis.

13. Topical pharmaceutical formulation comprising a composition according to any of claims 1 to 6 and pharmaceutical acceptable excipients.
